# EUROPEAN PATENT SPECIFICATION

(11) **EP 1 624 056 B1**
(45) Date of publication and mention of the grant of the patent: **19.11.2008**
(21) Application number: 05005743.9
(22) Date of filing: 16.03.2005
(51) Int. Cl.: C12N 9/20, C12N 1/20

(54) **Method for producing a recombinant thermostable Geobacillus T1 lipase**
Verfahren zur Herstellung einer rekombinanten thermostabilen Lipase von Geobacillus T1
Procède pour produir une lipase recombinante thermostable de Geobacillus T1

(30) Priority: 02.08.2004 MY 0403110
(43) Date of publication of application: 08.02.2006
(73) Proprietor: Universiti Putra Malaysia, Selangor (MY)
(72) Inventor: Rahman, Raja Noor Zaliha, 40400 Sha Alam, Selangor Darul Ehsan (MY); Salleh, Abu Bakar, 4300 Kajang, Selangor Darul Ehsan (MY); Basri, Mahiran, 22. Jalan LP 6/7, Selangor Darul Ehsan (MY); Chor, Leow Thean, Mk. Jerlun, 06150, Alor Setar, Kedah (MY)
(74) Representative: Krauss, Jan

(56) References cited:
- LEOW THEAN CHOR ET AL: "High level expression of thermostable lipase from Geobacillus sp. strain T1." BIOSCIENCE, BIOTECHNOLOGY, AND BIOCHEMISTRY. JAN 2004, vol. 68, no. 1, January 2004 (2004-01), pages 96-103, XP002354934 ISSN: 0916-8451
- HUANG XIAOHONG ET AL: "High-level expression and purification of a truncated merozoite antigen-2 of Babesia equi in Escherichia coli and its potential for immunodiagnosis." JOURNAL OF CLINICAL MICROBIOLOGY. MAR 2003, vol. 41, no. 3, March 2003 (2003-03), pages 1147-1151, XP002354935 ISSN: 0095-1137
- RAHMAN R N Z A ET AL: "Cloning and expression of a novel lipase gene from Bacillus sphaericus 205y." MOLECULAR GENETICS AND GENOMICS : MGG. MAY 2003, vol. 269, no. 2, May 2003 (2003-05), pages 252-260, XP002354936 ISSN: 1617-4615
- JAEGER KARL-ERICH ET AL: "Lipases for biotechnology." CURRENT OPINION IN BIOTECHNOLOGY. AUG 2002, vol. 13, no. 4, August 2002 (2002-08), pages 390-397, XP002354937 ISSN: 0958-1669
- RAHMAN R N Z R A ET AL: "Secretory expression of thermostable T1 lipase through bacteriocin release protein" PROTEIN EXPRESSION AND PURIFICATION, ACADEMIC PRESS, SAN DIEGO, CA, US, vol. 40, no. 2, April 2005 (2005-04), pages 411-416, XP004781394 ISSN: 1046-5928

## Description

### FIELD OF INVENTION

This invention relates to a method for producing a recombinant thermostable Geobacillus T1 lipase without the signal peptide (amino acids 1 to 28) as a GST-fusion protein in a prokaryotic host strain.

### BACKGROUND OF THE INVENTION

Lipase is a very important metabolic enzyme for common biological organisms; it can hydrolyze fat to produce free fatty acid. Lipases or acylglycerol hydrolases are enzymes that catalyse the hydrolysis of long chain triglyceride into diacylglyceride, monoglyceride, glycerol and free fatty acids. However, lipases are also capable of catalyzing the reverse reaction to hydrolysis in the formation of esters from alcohols and fatty acids, or through transesterification.

The lipase is extensively used as an enzyme for food processing in order to flavor dairy products, as medicament for improving digestion, for improving fats and oils, and the like. For each of these uses, the lipase is required to have various characteristics, and thermostable lipase is applied in order to achieve various characteristics for each use, and thermostable lipase is also variously applied in a large number of fields.

Microbial extracellular lipases are usually more thermostable than animal or plant lipases. Microbial extracellular lipase has a potential use in industries and diagnostics. A major requirement for a commercial enzyme is thermal stability, since thermal denaturation is a common cause of enzyme inactivation. In addition, increasing enzyme thermostability would allow enzymatic reactions to be carried out at higher temperature; this would help to increase conversion rates, substrate solubility and to reduce the possibility of microbial growth and the viscosity of the reaction medium.

Although thermophiles could be good candidates for producing thermostable enzyme, this is often impractical because of low yields, and also high temperature fermentation equipment may be needed. To overcome this problem, a molecular approach through genetic engineering is a good alternative in order to achieve high-level expression and to allow for the development of economic bulk production through the prokaryotic system. So far, several thermostable lipases have been successfully cloned and intracellularly expressed in heterologous hosts.

For fundamental studies and for commercial purposes, the expression of foreign proteins in prokaryotic systems is most widely used in order to achieve high-level expression. The expression vector and the host are important issues for achieving maximal expression of cloned gene, since the molecular cloning of a foreign gene does not ensure that said gene will be successfully expressed.

Huang Xiaohong et al. (in "High-level expression and purification of a truncated merozoite antigen-2 of Babesia equi in Escherichia coli and its potential for immunodiagnosis." Journal of Clinical Microbiology, March 2003, vol. 41, no. 3, pages 1147-1151) describe the expression level of the entire and truncated antigen, and only the truncated antigen is tested for the binding feasibility of fusion protein to affinity column. Therefore, the disclosure in Huang can not be fully applied to the present invention, which generally is directed to the interfering of the signal peptide with protein expression and purification. Huang furthermore focuses on the immunogenicity of the antigen as produced (which shall be used in ELISA assays), and not the functional enzyme, thus, proper functional folding is not 100% required. In doing so, in addition, the C terminus of the antigen is deleted.

Yoder et al. (in: "High-level expression of a truncated wall-associated protein A from the dental cariogenic Streptococcus mutans" DNA Cell Biol. 2000 Jul;19(7):401-8) explores on 12 truncated proteins regarding the expression level, and antibody cross-reactivity. However, the antibody cross-reactivity does not rely on proper functional folding as needed by T1 lipase for activity. The immunological activity of Yoder et al. is dependent only to some extend on protein folding. In addition, Yoder et al. did not further process the fusion protein to obtain any truncated protein without the tag.

Leung et al. (in: "Polyclonal antibodies to glutathione S-transferase--verotoxin subunit a fusion proteins neutralize verotoxins" Clin Diagn Lab Immunol. 2002 May;9(3):687-92) exclude the signal peptide (N-terminal) and trans-membrane (C-terminal) region for protein expression and are able to express large amount of soluble proteins. Leung et al. then attempt to cleave the GST from the fusion proteins before immunization, but the yields of the end product after thrombin cleavage was very low. No detailed procedure is discussed in Leung et al. regarding the removal of GST from the fusion protein.

In the present invention, a thermostable lipase that is rapidly cloned using Polymerase Chain Reaction (PCR) technique and manipulation of the thermostable lipase T1 gene is expressed through a prokaryotic system and subsequently purified.

### SUMMARY OF THE INVENTION

The present invention provides a method for producing a recombinant thermostable Geobacillus T1 lipase comprising a) expression of the thermostable Geobacillus T1 lipase without the signal peptide (amino acids 1 to 28) as a GST-fusion protein in a prokaryotic host strain, and b) affinity chromatography purification of said GST-fusion protein.

Preferred embodiments of the present invention are further described in the dependent claims.

### BRIEF DESCRIPTION OF THE DRAWINGS

Figure 1 is a diagram showing the micrograph of strain T1.
Figure 2 is a diagram showing the temperature activity of T1 mature lipase. Purified T1 lipase was assayed at different temperatures for 30 minutes under a shaking rate of 250 rpm with olive oil as substrate.
Figure 3 is a diagram showing an activity test of T1 lipase that was assayed at various pH for 30 minutes. T1 lipase was assayed at various pHs from pH 4 to pH 12 using a colorimetric method. Symbols used are: ◆ Acetate buffer; ▲, Potassium phosphate buffer; □, Tris-HCl buffer; ×, Glycine buffer; ○, Na₂HPO₄/NaOH buffer. For the stability test, the T1 lipase was assayed at 70°C for 30 min after pre-incubation at various pHs (1:1, v/v).
Figure 4 is a diagram showing the stability of T1 lipase that was assayed after pretreatment at various pHs from pH 4 to pH 12 for 30 minute. Then, the residual activity of T1 lipase was assayed after treatment with various pHs from pH 4 to pH 12 for 30 min. Symbols used are: ◆ Acetate buffer; ▲, Potassium phosphate buffer; □, Tris-HCl buffer; × , Glycine buffer; ○, Na₂HPO₄/NaOH buffer. For the stability test, the T1 lipase was assayed after pre incubation at various pHs (1:1, v/v) at 70°C for 30 min.
Figure 5 is a diagram showing the effect of metal ions on lipase activity.
Figure 6 is a diagram showing the effect of surfactants on lipase activity.
Figure 7 is a diagram showing the effect of substrates on lipase activity.
Figure 8 is a diagram showing the effect of inhibitors on lipase activity.

### DETAILED DESCRIPTION OF THE INVENTION

### Screening of Geobacillus sp. Strain T1

Isolation and identification of *Geobacillus* sp. Strain T1 that forms the basis of the present invention of thermophilic species: The *Geobacillus* sp. Strain T1 culture could be obtained from Enzyme and Microbial Technology Research Department of Biochemistry and Microbiology, Faculty of Science and Environmental Studies, University Putra Malaysia.

After the process of screening for a lipase producing bacterium from palm oil-mill effluent in Malaysia, the isolate T1, which gave positive results on triolein agar plates, was isolated. To verify the systematic position of this lipase-producing bacterium, a study of morphological and physiological characteristics, 16S rRNA analysis, cellular fatty acids analysis, DNA composition, DNA/DNA hybridization, and RiboPrint analysis was undertaken.

### Isolation and Identification of Geobacillus sp. Strain T1

Samples were taken from a palm oil-mill effluent in Selangor, Malaysia.

*Geobacillus* sp. Strain T1 was isolated from an enriched medium (pH 7.0) containing NaCl 0.2%,MgSO₄.7H₂O 0.04%, MgCl₂.6H₂O 0.07%, CaCl₂.2H₂O 0.05%, KH₂PO₄ 0.03%, K₂HPO₄ 0.03%, (NH₄)₂SO₄ 0.05%, with the sole carbon source of olive oil (2%), at 60°C under shaking conditions of 150 rpm. The strain was plated on triolein agar plate for screening as a lipase producer.

For morphological studies, pure bacterial strain was streaked onto a nutrient agar plate, and incubated at 60°C prior to Gram staining, the results were then observed under a light microscope. The morphological and physiological characteristics were sent to DSMZ (Deutsche Sammlung von Mikroorganismen und Zellkulturen) (German Collection of Microorganisms and Cell Cultures). The physiological characteristics study included catalase and oxidase test, and anaerobic growth, Voges-Proskauer test, growth at 30°C, 40°C and 70°C, growth in medium with pH 5.7, NaCl 2%, 5%, and lysozyme broth, fermentation of D-glucose, L-arabinose, D-xylose, D-mannitol and D-glucose, hydrolysis of starch, gelatin, casein and tween 80, decomposition of tyrosine, use of citrate and propionate, nitrate reduction, indol production, phenylalanine deaminase and arginine dihydrolase tests were performed. Fatty acids were extracted and analyzed following the instructions of the Sherlock microbial identification system.

The strain was furthermore deposited at the DSMZ on February 21, 2005 and received the Accession Number DSM 17139.

16S rDNA was amplified by PCR using two universal primers, and the PCR product was purified. A purified PCR product was cloned into TOPO TA PCR 2.1 cloning vector (Invitrogen). The recombinant plasmid was extracted, and then was sequenced using an ABI PRISM 377 DNA sequencer (Applied Biosystems).

The chromosomal DNA was isolated and purified and the G + C content was determined using chromatography conditions. The DNA was hydrolyzed, and the resulting nucleotides were analyzed by reverse-phase HPLC. DNA/DNA hybridization was carried out using a model 2600 spectrophotometer.

A standardized, automated ribotyping was performed using the Qualicon^{™} RiboPrinter system. The RiboPrinter system combines molecular processing steps for ribotyping in a stand-alone, automated instrument. The steps included cellular lysis, digestion of chromosomal DNA with the restriction enzyme *Eco*R1*,* separation of fragments by electrophoresis, transfer of DNA fragments to a nylon membrane, hybridization to a probe generated from the *rrn*B operon of *E.coli,* chemiluminescent detection of the probe as hybridized to the fragments containing *rrn* operon sequences, image detection and computer analysis of the RiboPrint patterns.

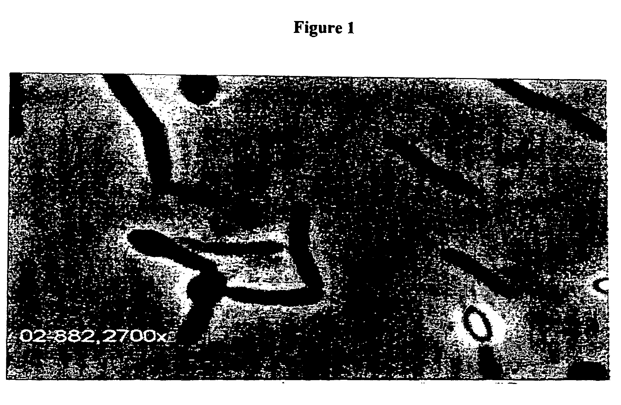

The isolated Strain T1 was an aerobic, gram-positive, endospore forming, rod-shaped bacteria (0.8-1.0 µm width and 2.5-6.0 µm long) (Figure 1). The DNA base composition of strain T1 was around 52.6% mol G + C. The cylindrical endospores appeared terminally in swollen sporangia. The growth of strain T1 occurred even at 70°C, but no growth was observed at 30°C and 40°C. The strain was tolerant to the presence of NaCl up to 2%. Acid was produced from D-fructose, but not from D-glucose and D-mannitol. The strain showed positive results both in citrate and nitrate tests. The morphological and physiological characteristics are presented in Table 1 and 2.

**Table 1. Morphological and physiological properties of Geobacillus strain T1.**

| Characteristic | *Geobacillus* strain T1 |
|---|---|
| Cell width (µm) | 0.8-1.0 |
| Cell length (µm) | 2.5-6.0 |
| Spores oval/cylindrical | + |
| Swollen sporangium | d |
| Catalase | + |
| Oxidase | - |
| Anaerobic growth | - |
| VP reaction | - |
| pH in VP broth | 4.9 |
| Growth at 30°C | - |
| 40°C | - |
| 70°C | + |
| | |
| Growth in | |
| Medium pH 5.7 | + |
| NaCl 2% | + |
| 5% | - |
| Lysozyme broth | - |
| | |

| Production of acid from: | |
|---|---|
| D-glucose | - |
| L-arabinose | w |
| D-xylose | w |
| D-mannitol | - |
| D-fructose | + |
| Gas from glucose | - |
| Lecithinase | n.g. |
| | |

| Hydrolysis of: | |
|---|---|
| Starch | + |
| Gelatin | - |
| Casein | - |
| Tween 80 | - |
| Decomposition of tyrosine | + |

| Use of: | |
|---|---|
| Citrate | + |
| Propionate | - |
| Nitrate reduction | + |
| Indole production | - |
| Phenylalanine deaminase | - |
| Arginine dihydrolase | - |

| | |
|---|---|
| + - positive; - - negative; w - weak reaction; n.d. - not determined; n.g. - no growth. | |

**Table 2. Comparison of biochemical, morphological and physiological properties of Geobacillus strain T1 and related thermophilic bacilli.**

| Characteristic | 1 | 2 | 3 |
|---|---|---|---|
| G + C mol% of DNA | 52.6 | 53.7 | 51.0 |
| Growth at 30°C | - | - | - |
| 70°C | + | + | + |
| Spore shape | C | O | O/C |
| Position | T | T | T |
| Catalase | + | + | n.d. |
| Oxidase | - | + | - |
| Anaerobic growth | - | - | - |
| Acid from glucose | - | + | + |
| Formation of acetoin | | - | - |
| Indole | - | - | - |
| H₂S | | n.d. | n.d. |
| Citrate utilization | + | - | + |
| Hydrolysis of: | | | |
| Starch | + | + | + |
| Gelatin | - | n.d. | + |
| Casein | - | W | - |
| Nitrate reduction | + | n.d. | + |

| | | | |
|---|---|---|---|
| + - positive; - - negative; w - weak reaction; n.d. - not determined; C - cylindrical; O - oval; T - terminal. Taxa are indicated as: 1, *Geobacillus zalihaii;* 2, *Geobacillus thermoleovorans* (Zarilla and Perry, 1986); 3, *Geobacillus kaustophilus* (White et al., 1993). | | | |

The complete 16S rDNA sequence of 1519 bp (AY166603) was determined as below.

1 gagtttgatc ctggctcagg acgaacgctg gcggcgtgcc taatacatgc aagtcgagcg
61 gaccggatcg gagcttgctc tgatttggtc agcggcggac gggtgagtaa cacgtgggca
121 acctgcccgc aagaccggga taactccggg aaaccggagc taataccgga taacaccgaa
181 gaccgcatgg tctttggttg aaaggcggcc tttggctgtc acttgcggat gggcccgcgg
241 cgcattagct agttggtgag gtaacggctc accaaggcga cgatgcgtag ccggcctgag
301 agggtgaccg gccacactgg gactgagaca cggcccagac tcctacggga ggcagcagta
361 gggaatcttc cgcaatgggc gaaagcctga cggagcgacg ccgcgtgagc gaagaaggcc
421 ttcgggtcgt aaagctctgt tgtgagggac gaaggagcgc cgttcgaaga gggcggcgcg
481 gtgacggtac ctcacgagga agccccggct aactacgtgc cagcagccgc ggtaatacgt
541 agggggcgag cgttgtccgg aattattggg cgtaaagcgc gcgcaggcgg ttccttaagt
601 ctgatgtgaa agcccacggc tcaaccgtgg agggtcattg gaaactgggg gacttgagtg
661 caggagagga gagcggaatt ccacgtgtag cggtgaaatg cgtagagatg tggaggaaca
721 ccagtggcga aggcggctct ctggcctgca actgacgctg aggcgcgaaa gcgtggggag
781 caaacaggat tagataccct ggtagtccac gccgtaaacg atgagtgcta agtgttagag
841 gggtcacacc ctttagtgct gcagctaacg cgataagcac tccgccgggg gagtacggcc
901 gcaaggctga aactcaaagg aattgacggg ggcccgcaca agcggtggag catgtggttt
961 aattcgaagc aacgcgaaga accttaccag gtcttgacat cccctgacaa cccaagagat
1021 tgggcgttcc cccttcgggg ggacaggtgg acaggtggtg catggttgtc gtcagctcgt
1081 gtcgtgagat gttgggttaa gtcccgcaac gagcgcaacc ctcgcctcta gttgccagca
1141 cgaaggtggg cactctagag ggactgccgg cgacaagtcg gaggaaggtg gggatgacgt
1201 caaatcatca tgccccttat gacctgggct acacacgtgc tacaatgggc ggtacaaagg
1261 gctgcgaacc cgcgaggggg agcgaatccc aaaaagccgc tctcagttcg gattgcaggc
1321 tgcaactcgc ctgcatgaag ccggaatcgc tagtaatcgc ggatcagcat gccgcggtga
1381 atacgttccc gggccttgta cacaccgccc gtcacaccac gagagcttgc aacacccgaa
1441 gtcggtgagg caacccgcaa gggagccagc cgccgaaggt ggggcaagtg attggggtga
1501 agtcgtaaca aggtagccg

The major amount of cellular fatty acids of strain T1 was iso-fatty acids (Table 3). Among these, from the iso-branched pentadecanoic acid (iso-C15), hexadecanoic acid (iso-C16) and heptadecanoic acid (iso-C17), which made up 78.33% of the total fatty acids, especially iso-C15 and iso-C17 were abundant. The fatty acid profile distinguishes the genus *Geobacillus* clearly from other mesophiles and thermophiles of the genera *Bacillus Alicyclobacillus, Brevibacillus, Aneurinibacillus, Sutfobacillus* and *Thermobacillus.* Although the strain T1 and *Geobacillus thermoleovorans* DSM 5366^{T} shared typical fatty acid profile of *Bacillus* rRNA group 5, it could be differentiated by the % composition of iso-C16, as indicated in Table 3.

**Table 3. Cellular fatty acids composition of Geobacillus strain T1 and its phylogenetic neighbor.**

| Fatty acid | *Geobacillus* sp. strain T1 | *Geobacillus* |
|---|---|---|
| | | *thermoleovorans* |
| | | (DSM 5366^{T}) |
| 10.0 | | 2.7 |
| 14.0 ISO | | 1.0 |
| 14:0 | 7.22 | 1.4 |
| 15:0 ISO | 32.42 | 22.6 |
| 15:0 ANTEISO | 1.01 | 1.3 |
| 15:0 | 0.82 | 2.1 |
| 16:0 ISO | 6.14 | 21.0 |
| 16:0 | 4.98 | 11.2 |
| 17:0 ISO | 39.77 | 18.5 |
| 17:0 ANTEISO | 4.97 | 4.6 |
| 17:0 | 0.53 | 1.3 |
| 18:1 ISO H | 0.38 | |
| 18:0 ISO | 0.36 | 0.9 |
| 18:0 | 0.47 | 3.4 |
| 18.1 | | 1.2 |
| 19:0 ISO | 0.91 | |
| unsaturated C 16 | | 6.6 |
| other | | 0.2 |

A DNA/DNA hybridization study was carried out in order to verify the taxonomic relationship between strain T1 and its phylogenetic neighbors. The genomic DNA/DNA, relation between strain T1 and type strains *Geobacillus kaustophilus* DSM 7263^{T} and *Geobacillus thermoleovorans* DSM 5366^{T} were 64.9 and 68.8, respectively (Table 4).

**Table 4: Levels of DNA/DNA re-association (%) among Geobacillus strain T1 and thermophilic bacilli.**

| Organisms | *Geobacillus* strain T1 |
|---|---|
| *Geobacillus kaustophilus* DSM 7263^{T} | 66.9; 62.9 (average = 64.9) |
| *Geobacillus thermoleovorans* DSM 5366^{T} | 70.0; 67.6 (average = 68.8) |

The DNA/DNA re-association values fell below the threshold value of 70% DNA/DNA similarity for the definition of species. The RiboPrint analysis was carried out for a decision about the affiliation of strain T1. However, the RiboPrint pattern of strain T1 was not identified by the Dupont identification library in order to identify it at the species level (>0.85). Its RiboPrint pattern showed the highest similarity to *Geobacillus kaustophilus* DSM 7263^{T} (0.69). The similarity to the pattern of *Geobacillus therleovorans* DSM 5366^{T} was somewhat lower (0.57). The pattern of *Geobacillus kaustophilus* DSM 7263^{T} and *Geobacillus thermoleovorans* DSM 5366^{T} show a binary similarity of 0.64.

As a consequence, the strain T1 was recognized as a member of a novel species through morphology and physiological studies, cellular fatty acids composition, DNA composition, DNA/DNA hybridization, and RiboPrint analysis. Therefore, we propose the creation of a novel species, *Geobacillus zalihae* sp. nov., for strain T1^{T}. (za.li.'hae N.L. gen. n. zalihae of zaliha, in honor to the scientist from Universiti Putra Malaysia, who has contributed significantly to extremophiles). The cells are rod-shaped, 0.8-1.0 width and 2.5-6.0 length, gram positive bacteria. The terminal spores are cylindrical and swollen in the sporangium. The DNA base composition of strain T1 is around 52.6% mol G + C. It contains a major amount of iso-fatty acids with iso-C15, and iso-C17 in abundance (77.19%). Growth is aerobic and the strain still growths at 70°C and is tolerant to up to 2% NaCl. It cannot perform anaerobic growth. Catalase and nitrate tests are positive. No acid is formed from D-glucose and D-mannitol. The strain can utilize citrate. Starch is hydrolyzed, but not gelatin and casein.

### Cloning and Sequencing of thermostable lipase gene from Geobacillus sp. Strain T1 Bacterial strains and Plasmids

Recombinant plasmid pBAD/T1 (Leow *et al.,* 2004) carrying the thermostable T1 lipase gene was used as source for subcloning. *E.coli* strains were grown in LB medium at 37°C. pRSET C (Invitrogen), pET22b(+) (Novagen) and pGEX-4T1 (Amersham Bioscience; United Kingdom, England) were used for subcloning and expression.

### DNA manipulation

The plasmid DNA was isolated with a QIAGEN miniprep spin kit (QIAGEN; Hilden, Germany) according to the manufacturer's instructions. The PCR product was purified with a GeneClean Kit (Qbiogene; Carlsbad, USA) as described by the supplier. Competent cells of *E. coli* were prepared by using a conventional CaCl₂ method.

### Subcloning and Expression of the thermostable T1 lipase gene.

Subcloning of the T1 lipase gene was done by designing a set of primers which incorporated restriction enzyme sites *Bam*H1/*Eco*R1 which included primers pGEX-For 5'-GAA GGG ATC CGT GAT GAA ATG CTG TCG GAT TAT G-3' and pGEX -Rev : 5'-AAT AGA ATT CTT AAG GCT GCA AGC TCG CCA A-3' for subcloning of the open reading frame of T1 lipase, and EH2-F : 5'-GAC GGG ATC CGC ATC CCT ACG CGC CAA TGA T-3' and pGEX - Rev. 5'-AAT AGA ATT CTT AAG GCT GCA AGC TCG CCA A-3' for subcloning of the T1 mature lipase gene. The ligated plasmid was used to transform *E. coli* strains and screened with tributyrin LB agar plates with appropriate antibiotics. E. coli BL21(De3)plysS harboring recombinant plasmids were grown in 1L blue cap bottles containing 200 ml of LB medium, supplemented with 100 µg/ml ampicillin and 35 µg/ml chloramphenicol on a rotary shaker (200 rpm) at 37°C.

The recombinant clones with and without signal peptide were induced with 1 mM of isopropyl-β-D-thiogalactopyranoside (IPTG) at OD₆₀₀ₙₘ ~ 0.5 for different induction periods (0, 4, 8, 12, 20, 28, 36 and 44 h). The induction was optimized further by varying the concentration of IPTG (w/o, 0.025, 0.05, 0.1, 0.5, 1.0, 1.5, and 2.0 mM) and the induction OD₆₀₀ₙₘ (0.25, 0.50, 0.75 and 1.00). Cultures (10 ml) were harvested by centrifugation and resuspended with 2 ml of 50 mM of potassium phosphate buffer (pH 7.0) before sonication (Branson 250 sonifier: output 2, duty cycle 30 and min 2) and cleared by centrifugation (12,000 rpm, 20 min). The clear crude lysate was used for the lipase assay.

### Electrophoresis

SDS-PAGE that was done on 12% of gel was used to analyze the protein. A broad range of protein standards (MBI Fermentas; St. Leon-Rot, Germany) was used as a molecular mass marker.

### Purification of T1 lipase

Four hundred ml of recombinant culture were harvested by centrifugation and concentrated to 40 ml prior to sonication. The crude cell lysate was loaded on a glutathione-Sepharose HP column (XK 16/20) with a 10 ml column volume equilibrated with PBS (pH 7.3), at a flow rate of 0.2 ml/minute. The column was washed with the same buffer until no protein was detected. The bound lipase was eluted with thrombin cleavage buffer (20 mM Tris-HCl, 100 ml NaCl and 0.33 mM CaCl₂, pH 8.4 supplemented with 10 mM reduced glutathione. The fusion protein was subjected to thrombin cleavage at 20°C for 20 h, and the buffer was exchanged with Sephadex G-25 prior to dialysis against PBS (pH 7.3). The GST tag and the thrombin enzyme were further removed using Glutahione-Sepharose HP, HiTrap Glutathione 4FF and HiTrap Benzamidine in series.

The expression of T1 lipase including the signal peptide was achieved using a prokaryotic system involving pBAD, pRSET C, pET22b(+) and pGEX-4T1 which were under the control of *araC,* T7, T7 *lac* and *tac* promoters, respectively. Among them, a relatively higher level of expression was achieved with the pGEX-4T1 expression system which is under the control of *tac* promoter for an inducible high level expression. This was chosen for further study. Simple optimization was carried out in order to increase the soluble protein of a recombinant clone harboring recombinant plasmid pGEX-4T1 with signal peptide. A total of 11,708 U of lipase activity was detected when induced with 0.05 mM of IPTG at OD₆₀₀ₙₘ ~0.5 for 8 hours, which corresponded to a specific activity of 30.192 U/mg. (Table 5).

**Table 5: Expression of the T1 lipase gene with and without signal peptide.**

| Optimization condition | Total activity from 1L culture (U) | |
|---|---|---|
| | Signal peptide | Without signal peptide |
| Induction time (h) | 5,627 (8) | 23,915 (12) |
| Inducer concentration (mM) | 11,708 (0.050) | 31,609 (0.025) |
| Induction OD₆₀₀ₙₘ | - | 41,902 (0.750) |

| | | |
|---|---|---|
| Note: The host BL21(De3)plysS, harboring recombinant plasmid pGE3C/T1 and pGEX/T1S was induced with 1 mM of IPTG at OD₆₀₀ₙₘ ~ 0.5 for different times. The bracket indicates the optimum values of every parameter. The total activity was calculated based on 1L of culture. | | |

Theoretically, the expression of T1 lipase as GST fusion protein in prokaryotic system allows for a rapid purification of recombinant lipase through affinity chromatography. However, we failed to purify the soluble fraction of recombinant lipase, because it did not bind to glutathione Sepharose 4FF, even though a high level of activity was detected.

This might be due to high hydrophobic regions of GST and the signal peptide that lead to conformation changes and improper folding of the GST domain. In some cases, GST fusion proteins are totally or partly insoluble. Furthermore, high level expression might be contributing to improper folding of partial and highly insoluble fusion protein and subsequently interfere with binding to the affinity column.

According to Frangioni and Neel (1993), the insolubility of GST fusion protein is common. Therefore, the sarcosyl lysis method was used in order to solubilize partially improper folded active T1 fusion lipase. The non-ionic detergent Triton X-100 was used in order to sequester sarcosyl with the hope to refold back the solubilized fusion protein in a correct folding, which subsequently allows the GST fusion protein to bind with affinity glutathione Sepharose 4FF. As shown in Table 6, a recovery of around 25% and purification fold of 7.3 was achieved through anionic and non-ionic detergent-treatment of the fusion protein.

**Table 6: Purification of GST fusion lipase with signal peptide**

| Purification | Total activity | Total protein | Specific activity | Recovery | Purification |
|---|---|---|---|---|---|
| steps | (U) | (mg) | (U/mg) | (%) | fold |
| Cell lysate | 1,824.90 | 37.90 | 48.15 | 100.00 | 1.00 |
| Glutathione Sepharose | 454.79 | 1.29 | 352.55 | 24.92 | 7.31 |

| | | | | | |
|---|---|---|---|---|---|
| Note: The fusion protein was solubilized with 2% anionic detergent sarcosyl and 1% non-ionic detergent Triton X-100 for binding in the affinity chromatography. | | | | | |

The signal peptide controls the entry of virtually all proteins into the secretory pathway, both in eukaryotes and prokaryotes, and is cleaved off while the protein is translocated through the membrane. However, it does not contribute to the structural gene of the thermostable T1 lipase. Thus, we tried to express the T1 lipase by omitting the signal peptide with the aim to improve the folding and interaction of the GST tag and the T1 mature lipase, without interfering with the affinity binding of the GST fusion protein to the immobilized glutathione on the matrix support.

The removal of signal peptide rigidifies the fusion lipase, since the removal of a total of 28 amino acid residues limited the movement of T1 lipase in covering the GST moiety. Manipulation of T1 lipase gene significantly improved the expression level by 4.25 and 2.70 fold for induction time and inducer concentration, respectively (Table 5). Further optimization of the expression achieved an expression level of 41,902 U/L of culture when induced with 0.025 mM of IPTG at OD₆₀₀ₙₘ~0.75 for 12 hours. There was an around 279 fold increase in expression level, when compared to wild-type *Geobacillus* sp. Strain T1.

Removing the signal peptide from the fusion protein not only improved the yield of active T1 lipase, but also simplified the purification using affinity chromatography in glutathione Sepharose 4FF column to homogeneity. As shown in Table 7, the recovery rate was around 72.55% with the purification fold being 2.87. Purification of fusion lipase increased the specific activity from 103.762 U/mg (crude cell lysate) to 297.929 U/mg (purified fusion lipase).

T1 mature lipase was obtained through subsequent purification steps (Table 8). The fusion lipase was cleaved with thrombin protease at 20°C for 20 hours and subjected to Sephadex G-25 in order to exchange the buffer to PBS (pH 7.3) and to remove free glutathione prior to dialysis. The lipase was subjected to affinity chromatography in glutathione sepharose HP, HiTrap glutathione sepharose 4FF and Benzamidine FF (high sub) in series, in order to remove the GST tag and the thrombin enzyme.

**Table 7: Purification of GST fusion lipase without signal peptide**

| Purification | Total activity | Total protein | Specific activity | Recovery | Purification |
|---|---|---|---|---|---|
| Steps | (U) | (mg) | (U/mg) | (%) | fold |
| Cell lysate | 3,732.331 | 35.970 | 103.762 | 100.00 | 1.00 |
| Glutathione Sepharose | 2,707.880 | 9.089 | 297.929 | 72.55 | 2.87 |

| | | | | | |
|---|---|---|---|---|---|
| Note: Direct purification of fusion protein was conducted with a Glutathione Sepharose 4FF affinity column without any detergent. | | | | | |

**Table 8: Purification of T1 mature lipase**

| Purification | Total activity | Total protein | Specific activity | Recovery | Purification |
|---|---|---|---|---|---|
| Steps | (U) | (mg) | (U/mg) | (%) | fold |
| Cell lysate | 28,848.120 | 138.255 | 208.659 | 1.00 | 100.00 |
| Affinity 1 | 20,557.051 | 42.038 | 489.011 | 71.26 | 2.34 |
| Affinity 2 | 14, 852.742 | 15.487 | 959.03 | 51.49 | 4.60 |

| | | | | | |
|---|---|---|---|---|---|
| Note: Purification of T1 mature lipase was conducted with affinity chromatography. Affinity 1 represents glutathione Sepharose HP. Affinity 2 represents glutathione Sepharose HP, HiTrap glutathione Sepharose 4FF and Hitrap Benzamidine (high sub) arranged in series | | | | | |

### Characterization of T1 fusion and mature lipase

The T1 lipase was tested at temperatures ranging from 40°C to 80°C at 5°C intervals for 30 min for enzyme activity. The thermostability test was conducted at various temperatures for different numbers of hours. Recombinant T1 lipase was also kept at a wide range of pH values ranging from pH 4-12 for pH activity and stability determination. The buffer systems used were 50 mM acetate buffer (pH 4-6), potassium phosphate buffer (pH 6-8), Tris-HCl buffer (pH 8-9), glycine-NaOH buffer (pH 9-11), and Na₂HPO₃/NaOH buffer (pH 11-12). The effects of effectors (metal ions, surfactants) and inhibitors were studied at a concentrations of 1 mM and 5 mM, respectively at 50°C for 30 min, with the exception of pepstatin (1.0 mM).

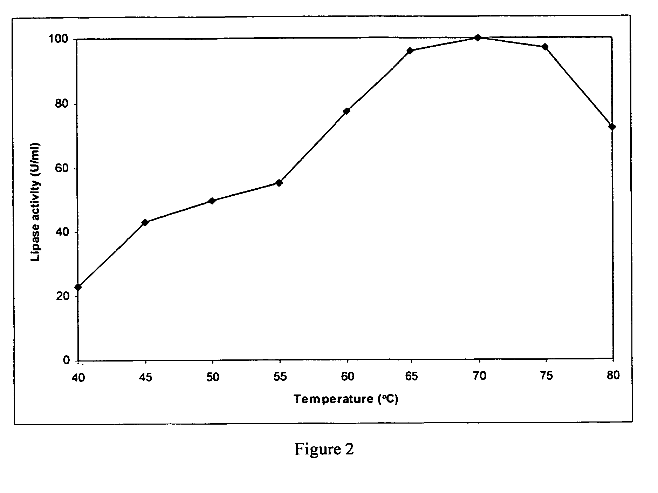

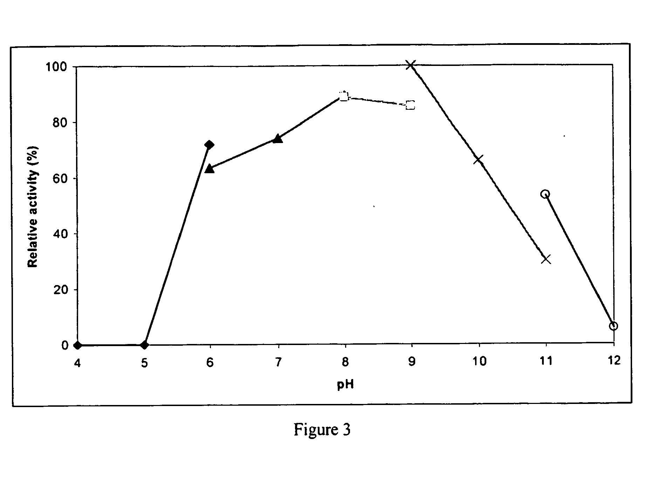

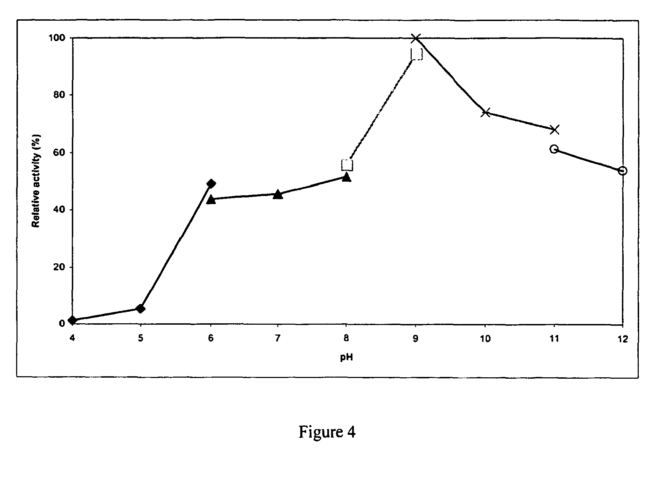

The effect of temperature activity and stability was tested at various temperatures for 30 min. The enzyme was most active in the temperature range of 60°C to 80°C, with maximal activity at 70°C (Figure 2). The effect of pH on the activity and stability of the lipase activity was studied at various pHs for 30 min under shaking condition. As shown in Figure 3, the lipase was active over a wide pH range, especially at pH 6 to 10, with an optimum pH of 9, with olive oil as substrate. The pH treatment showed that T1 mature lipase was relatively stable at alkaline pH (Figure 4).

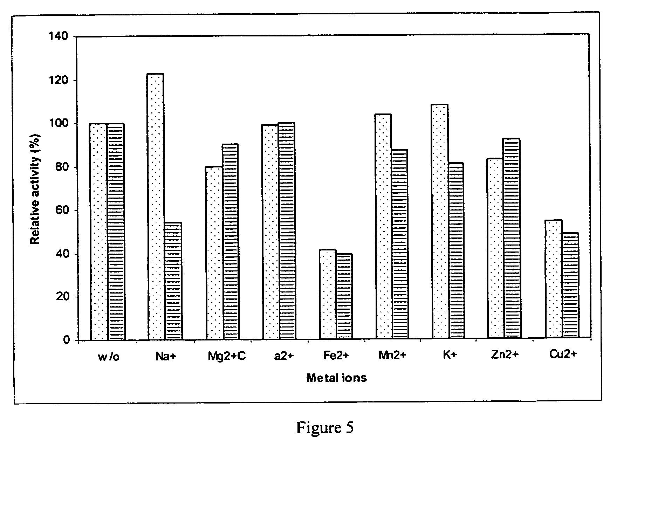

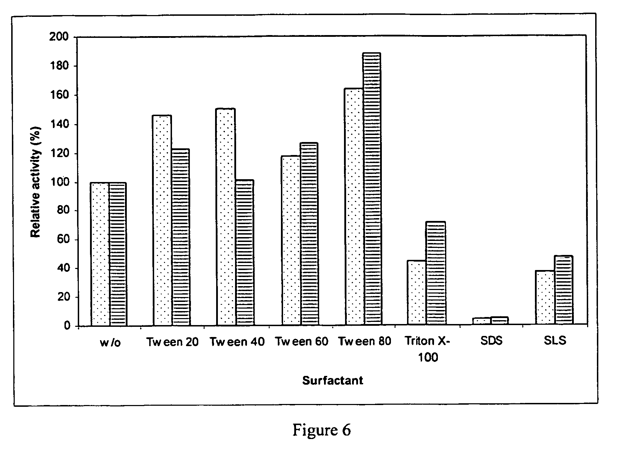

The effect of metal ions at a final concentration of 1 mM was tested through treatment at 50°C for 30 min. As shown in Figure 5, Na⁺, Mn²⁺ and K⁺ slightly enhanced the lipase activity after 15 min of treatment. Mg²⁺, Mn²⁺, Ca²⁺, K⁺ and Zn²⁺ showed stabilizing effects even after 30 min of treatment, in particular Ca²⁺, without a significant loss of enzyme activity. However, Fe²⁺ and Cu²⁺ strongly inhibited the lipase activity by 60% and 52% after 30 min. The effect of surfactants on lipase activity was studied at a concentration of 0.1% (Figure 6). Upon the addition of 0.1% Tween, an obvious enhancement was seen, in particular for Tween 80, which increased the lipase activity by 63% and 88% after 0 and 30 min of treatment, respectively.

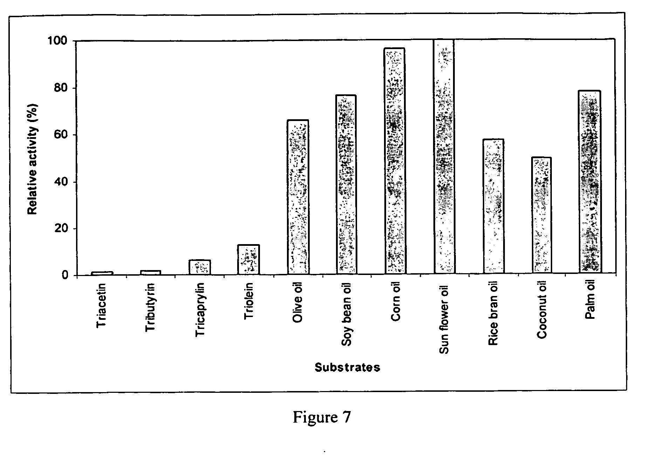

The effect of substrates on lipase activity of various triglycerides and natural oils was used to study the substrate specificity. Figure 7 shows that the T1 lipase prefers natural oils as substrates, compared to triglycerides. It is more selective towards long carbon chain natural oils such as olive oil, corn oil, sunflower oil and palm oil. This enables the use of T1 lipase as catalyst in industrial application.

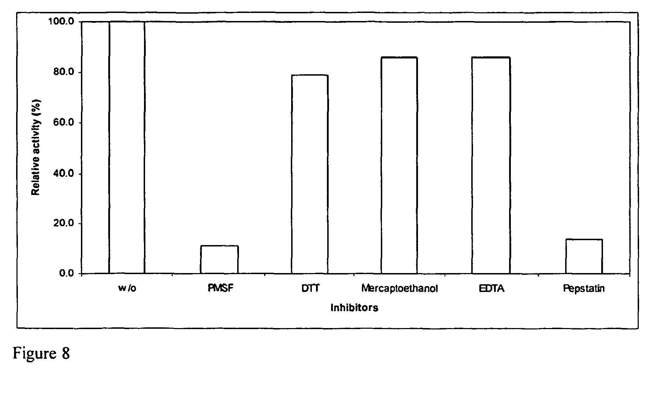

The inhibitory effects on lipase activity of several metal chelating agent, reducing agents, serine and aspartate inhibitors were used to study the inhibitory effect against T1 mature lipase. It is not a metalloenzyme, since EDTA showed only a slight effect on lipase activity even at 5 mM. The reducing agents, such as β-mercaptoethanol and DTT gave little inhibitory effect on T1 lipase. The lipase was strongly inhibited by the addition of 5 mM PMSF and 1 mM of Pepstatin, showing that serine and aspartate residues play an important key role in the catalytic mechanism (Figure 8). The P1 lipase from *Bacillus stearothermophilus* showed 77% inhibition in the presence of 10 mM of PMSF (Sunchaikul et al., 2001).

The properties of purified T1 fusion and mature lipase were compared in order to study the effect of the GST tag on lipase activity. Both lipases was purified and their physiochemical properties were studied (Table 9). The fusion partner slightly reduced the optimal temperature and pH to 65°C and pH 8, respectively. No significant difference was observed with metal ions and substrates. However, Tween 20-80 enhanced the effect of T1 mature lipase but were only stable in the presence of fusion partners. Both of them were inhibited by serine and aspartate inhibitors at concentrations as tested.

**Table 9: Comparison of thermostable T1 mature lipase and fusion lipase**

| Properties | Fusion lipase | Mature lipase |
|---|---|---|
| Optimum temperature | 65°C | 70°C |
| Temperature stability | T_{1/2} = 2.5h at 65°C | T1_{/2} = 12 h at 60°C |
| Optimum pH | 8 | 9 |
| pH stability | 8-10 | 8-11 |
| Metal ions | Mg²⁺, Ca²⁺, Mn²⁺, K⁺ and Na⁺ | Mg²⁺, Ca²⁺, Mn²⁺, K⁺ and Zn²⁺ |
| Surfactants | Stable with Tween and Triton X-100 | Stable with Tween and Triton X-100 |
| Substrates | natural oils | natural oils |
| Inhibitors | PMSF and Pepstatin | PMSF and Pepstatin |

Since the GST tag was rigidly fused to the T1 mature lipase without a signal peptide, it only conferred a minimal effect on the tertiary structure and biological activity, without significantly changing physiocochemical properties. There are no significant differences between the T1 fusion and the mature lipases except for the optimum pH and temperature, wherein only one unit pH and 5°C higher was observed for T1 mature lipase. The behavior of T1 fusion and mature lipase to serine and aspartate inhibitors was the same. Therefore, we proposed the T1 lipase fused to GST tag for a higher economical production, to simplify fusion protein purification for industrial application especially for detergent formulations for warm wash laundry detergent. In addition, the precipitate formed by the fusion protein because of aggregation at high concentrations of fusion lipase was readily solubilized by resolving it in buffer of pH 9 that is far away from the pI of the fusion lipase.

## Claims

1. A method for producing a recombinant thermostable Geobacillus T1 lipase comprising
a) expression of the thermostable Geobacillus T1 lipase without the signal peptide (amino acids 1 to 28) as a GST-fusion protein in a prokaryotic host strain, and
b) affinity chromatography purification of said GST-fusion protein.

2. The method according to claim 1, further comprising
c) cleaving off the GST-tag from said GST-fusion protein.

3. The method according to claim 2, further comprising
d) further purification of said recombinant thermostable Geobacillus T1 lipase

4. The method according to any of claims 1 to 3, wherein no detergent is used for the purification steps.

5. The method according to any of claims 1 to 4, wherein the recombinant thermostable T1 lipase is isolated from Geobacillus Strain T1-DSM 17139.

## Patentansprüche

1. Verfahren zur Herstellung einer rekombinanten thermostabilen Geobacillus T1 -Lipase, umfassend
a) Expression der thermostabilen Geobacillus T1-Lipase ohne das Signalpeptid (Aminosäuren 1 bis 28) als ein GST-Fusionsprotein in einem prokaryontischen Wirtsstamm, und
b) Reinigung des GST-Fusionsproteins mittels Affinitäts-Chromatographie.

2. Verfahren nach Anspruch 1, weiter umfassend
c) Abspalten des GST-Anteils von dem GST-Fusionsprotein.

3. Verfahren nach Anspruch 2, weiter umfassend
d) weitere Reinigung der rekombinanten thermostabilen Geobacillus T1-Lipase.

4. Verfahren nach einem der Ansprüche 1 bis 3, wobei für die Reinigungsschritte kein Detergens verwendet wird.

5. Verfahren nach einem der Ansprüche 1 bis 4, wobei die rekombinante thermostabile T1-Lipase aus dem Geobacillus Stamm T1-DSM 17139 isoliert wird.

## Revendications

1. Procédé de production d'une lipase recombinante thermostable de Geobacillus T1 comprenant
a) l'expression de la lipase thermostable de Geobacillus T1 sans le peptide de signal (acides aminés 1 à 28) comme protéine de fusion GST dans une souche hôte procaryote, et
b) la purification par chromatographie d'affinité de ladite protéine de fusion GST.

2. Procédé selon la revendication 1, comprenant en outre
c) le clivage de GST-tag de ladite protéine de fusion GST.

3. Procédé selon la revendication 2, comprenant en outre
d) une nouvelle purification de ladite lipase recombinante thermostable de Geobacillus T1.

4. Procédé selon l'une quelconque des revendications 1 à 3, dans lequel on n'utilise aucun détergent pour les étapes de purification.

5. Procédé selon l'une quelconque des revendications 1 à 4, dans lequel la lipase T1 recombinante thermostable est isolée à partir de la souche de Geobacillus T1-DSM 17139.
